# EUROPEAN PATENT APPLICATION

(11) **EP 1 745 817 A1**
(43) Date of publication of application: **24.01.2007**
(21) Application number: 05425516.1
(22) Date of filing: 19.07.2005
(51) Int. Cl.: A61N 1/16

(54) **Device for modifying and rebalancing ionisation for an electrical load**

(71) Applicant: Iero, Demetrio Paolo, 89100 Reggio Calabria (IT); Di Loreto, Giuseppe, 00049 Velletri (IT); Pesante, Adriana, 00044 Frascati (IT)
(72) Inventor: Iero, Demetrio Paolo, 89100 Reggio Calabria (IT); Di Loreto, Giuseppe, 00049 Velletri (IT); Pesante, Adriana, 00044 Frascati (IT)
(74) Representative: Cerbaro, Elena

(57) **Abstract**

A device for modifying and rebalancing ionisation (1), in particular of air and biological matter, applicable to an electrical load (5) powered by an electrical power source (3); the device (1) is provided with a pair of inputs (2) designed to be connected to the electrical power source (3), a pair of outputs (4) designed to be connected to the electrical load (5), and at least one pair of electrical conductive elements (6) mutually crossingly arranged and electrically insulated one from the other so that each is extended between an input (2) and a respective output (4) and is crossed by at least one part of an electrical current (IA) absorbed by the electrical load (5).

## Description

The present invention relates to a device for modifying and rebalancing ionisation for an electrical load.

In particular, the present invention finds advantageous, although not exclusive, application in an electrical load of the domestic or industrial type in order to modify and rebalance the ionisation of air and biological matter in presence of an electromagnetic field produced by the electrical load itself, to which the following description refers without for this losing in generality.

It is known that air ionisation, that is the concentration of positive and negative ions in gaseous form in the air, influences the biological activity of the cells in living organisms. In particular, it is scientifically acknowledged that when a living organism, either human, animal or vegetable, remains exposed for a long time to a high percentage of positive ions, with respect to a natural comprehensive concentration of negative and positive ions, it undergoes metabolic alterations, for example an alteration of the sodium-potassium pump operation that governs the so-called "cellular respiration" function, feeding, in the specific case of the human and animal organism, a series of muscular, dermatological, respiratory, rheumatic, cardiologic, psychiatric and psychological pathologies.

In the air of natural outdoor environments, as in the mountains or in the open countryside, there are normally present high concentration of negative ions and low concentrations of positive ions.

In closed environments, instead, the concentration of both positive and negative ions is typically lower, due to the poor exchange of fresh air, and the percentage of positive ions may also be more than double with respect to the percentage of negative ions. Indeed, buildings of the modern type often contain metallic structures and coverings of synthetic type, on which are accumulated positive electrostatic charges capable of absorbing a large amount of negative ions. Furthermore, it is experimentally demonstrated that alternating electromagnetic fields generated by electrical loads for domestic or industrial purposes, combining with the terrestrial magnetic field, unfavourably modify the concentration ratio of positive and negative ions. More precisely, considering a vectorial representation of the magnetic fields, the vector associated to the magnetic component of the alternating electromagnetic field generated by an electrical load arranges parallelly to the vector associated to the terrestrial static magnetic field, favouring the formation of positive ions.

Generally, the excess of positive ions in a closed environment is solved with the use of specific ionisation apparatuses capable of outputting electrons which negatively charge the molecules of oxygen and nitrogen present in the air thus forming negative ions.

However, this solution is disadvantageous from an economic point of view, because typically one ionisation device must be used for each closed environment. Furthermore, a possible centralised ionisation system which produces and distributes, by means of specific pipings, negative ions to several environments would present the further drawback of producing an accumulation of electrostatic positive charges along the walls of the pipings caused by the flow of the air along the pipings themselves, which accumulation causes a corresponding absorption of a large amount of the produced negative ions.

It is likewise scientifically demonstrated that the exposure of biological matter belonging, for example, to a human, animal or vegetable organism, to a combination of parallel magnetic fields of weak intensity, for example the combination between the terrestrial magnetic field and the magnetic component of the alternating electromagnetic field generated by an electrical load which is in the vicinity of the biological matter, may determine a transient variation of the flow of various biological ionic species through the cellular membranes of the biological matter itself, altering in general the metabolism of the organism to which the biological matter belongs.

It is the object of the present invention to provide a device for modifying and rebalancing ionisation, in particular of air and biological matter, applicable to any electrical or electronic load, which device is free from the aforementioned drawbacks and, at the same time, is easy and cheap to make.

According to the present invention, there is provided a device for modifying and rebalancing ionisation for an electrical load as recited in the accompanying claims.

For a better understanding of the present invention, there will now be described preferred embodiments, only by way of non-limitative example and with reference to the accompanying drawings, in which:
- figure 1 schematically shows a first preferred embodiment of the device for modifying and rebalancing ionisation according to the present invention;
- figure 2 schematically shows a second preferred embodiment of the device for modifying and rebalancing ionisation according to the present invention;
- figure 3 schematically shows a third preferred embodiment of the device for modifying and rebalancing ionisation according to the present invention; and
- figure 4 shows, according to a perspective exploded view, an embodiment of a detail of the device for modifying and rebalancing ionisation of figure 3.

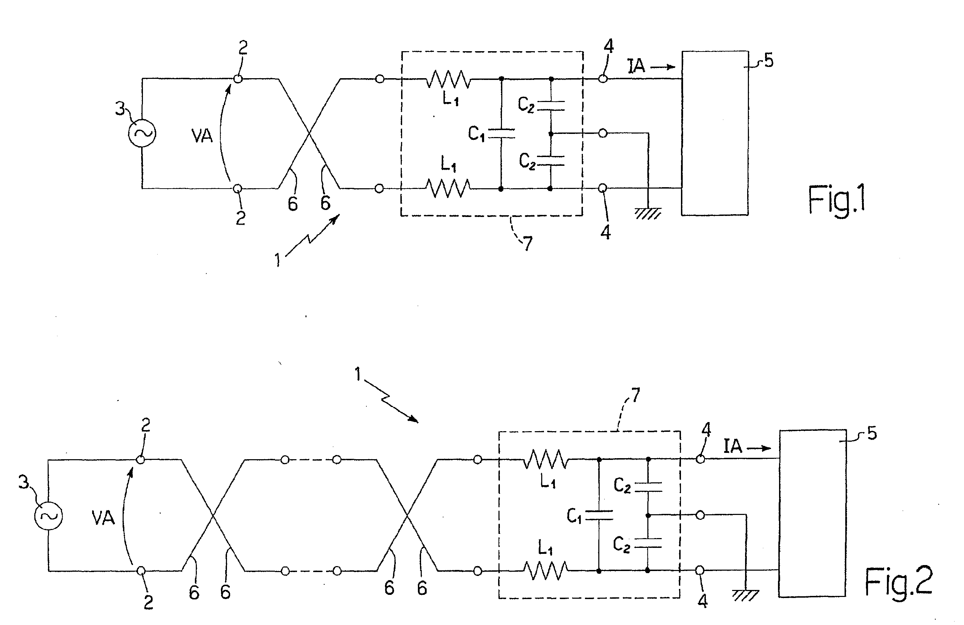

In figure 1, number 1 indicates, by means of a principle electric diagram, a first preferred embodiment of a device for modifying and rebalancing ionisation comprising a pair of inputs 2 designed to be connected to an electrical power source 3; a pair of outputs 4 designed to be connected to an electrical load 5; a pair of electrical conductive elements 6 mutually crossingly arranged without reciprocal contact, or by interposing between them an electrically insulating material (not shown), and in such a manner that each of the two electrical conductive elements 6 extends between an input 2 and a respective output 4; and an electrical filter 7 of the passive type connected between the pair of electrical conductive elements 6 and the pair of outputs 4 to remove interference signals from the electrical energy source 3.

For electrical load 5 it is hereinafter intended a generic electrical load consisting, for example, of a household appliance, an electrical tool, a television set, a cellular telephone or any other apparatus which needs alternating or direct electrical power to operate, or of a combination in series or in parallel of a plurality of the aforementioned apparatuses. Consequently, the electrical power source 3 is of the alternating or, respectively, direct type, and the electrical filter 7 is of the type operating on alternating current or on direct current, respectively.

The electrical conductive elements 6 are made of a same electrical conductive material, for example metal, metallic alloy, superconductor material, conducting rubber or the like, or by two different electrical conductive materials, for example two different metals or metallic alloys, or superconductor materials. The value of the section area of the electrical conductive elements 6 depends on a nominal electrical power value absorbed by the electrical load 5. Furthermore, the electrical conductive elements 6 preferably, but not necessarily, present a straight shape and define, by mutually crossing, any angle.

As shown in figure 1, the device 1 is applied to an electrical load 5 operating on alternating current and the electrical filter 7 consists of a passband double-n filter dimensioned to operate at a frequency from 50 to 60 Hz, at a maximum electrical voltage amplitude value equal to 240 V and a maximum current amplitude value equal to 6 A. In particular, the electrical filter 7 comprises a pair of inductances L1, each equal to 2.1 mH, a capacitance C1 equal to 0.1 µF (350 V of maximum applicable voltage), and a pair of capacitances C2, each equal to 3300 pF (350 V of maximum applicable voltage).

In actual use, the electrical power source 3 applies an alternating electrical voltage VA on the inputs 2 of the device 1, which is consequently crossed by at least one part of an alternating electrical current IA absorbed by the electrical load 5 and depending on the nominal electrical power absorbed by the electrical load 5. The electrical conductive elements 6, while they are crossed by said part of the electrical current IA, reverse, due to their crossed arrangement, the electrical voltage present at the input of the electrical filter 7 with respect to that applied to the pair of inputs 2, so as to prevent the vector of the magnetic component of the alternating electromagnetic field generated by the electrical load 5 from arranging itself parallelly to the vector of the terrestrial magnetic field.

Assuming that the electrical load 5 and the device 1 connected thereto are operated in a closed environment, in the air present in such environment it is experimentally observed a decrease of positive ions with respect to the case of absence of the device 1, which, therefore, is capable of modifying and rebalancing the ionisation of the air in the closed environment towards a concentration ratio of positive and negative ions more similar to that which characterises a natural outdoor environment.

Furthermore, it is experimentally observed that the device 1, when applied to the operating electrical load 5, is capable of limiting the transient variations of the flows of certain ionic species through the cellular membranes of biological matter which is in the vicinity of the electrical load 5, thus rebalancing the ionisation of the biological matter with respect to the case of absence of the device 1.

Figure 2 shows a second preferred embodiment of the device 1 which differs from the first preferred embodiment shown in figure 1 in that it comprises a plurality of said pairs of electrical conductive elements 6 mutually cascadingly connected between the inputs 2 and the outputs 4. In other words, each electrical conductive element 6 of each upstream pair is electrically connected in series to a respective electrical conductive element 6 of the downstream pair so that each pair of electrical conductive elements 6 is crossed, in use, by said part of electrical current IA.

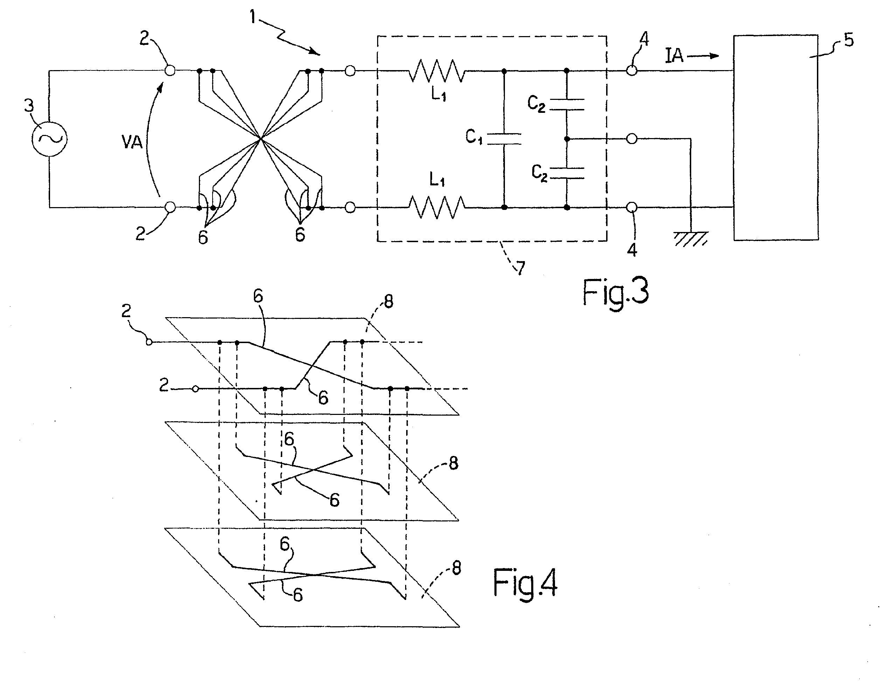

Figure 3 shows a third preferred embodiment of the device 1 which differs from the first preferred embodiment shown in figure 1 in that it comprises a plurality of said electrical conductive elements 6, and in particular three pairs of electrical conductive elements 6, mutually electrically connected in parallel between the inputs 2 and the outputs 4 so as to be crossed, in use, by respective fractions of said part of the electrical current IA.

In particular, with reference to the exploded perspective view shown in figure 4, the pairs of electrical conductive elements 6 are ideally arranged on respective mutually parallel and electrically isolated planes 8 according to a "wafer" structure and the two electrical conductive elements 6 of each pair define, by mutually crossing, a relative angle. The angles defined by the pairs of electrical conductive elements 6 assume respective mutually different values and are determined so as to remove as much as possible the magnetic component of the electromagnetic field generated by the electrical load 5 from the abovementioned condition of parallelism with the terrestrial magnetic field.

A fourth preferred embodiment of the device 1 (not shown) differs from the embodiments previously described and shown in figures 1, 2, 3 and 4 in that the electrical filter 7 is connected between the pair of inputs 2 and the electrical conductive elements 6.

A fifth embodiment of the device 1 (not shown) differs from the previously described embodiments in that the electrical filter 7 is of the active type. Such type of electrical filter 7 avoids that possible interference signals produced by the electrical load 5 are transmitted to the electrical power source 3.

The main advantage of all the embodiments of the device 1 described above, when connected to an electrical load 5 in operation, is in limiting at the source the formation of positive ions in the air present inside a closed environment in which the electrical load 5 itself is located, modifying and rebalancing, in such a manner, the ionisation of the air in the closed environment towards a concentration ratio of positive and negative ions more similar to that which characterises a natural outdoor environment.

A further advantage of the device 1 described above is to limit the transient variations of the flows of certain ionic species through the cellular membranes of biological matter in a living organism which is found in the vicinity of the operating electrical load 5, limiting the alterations of the metabolism of said living organism.

Furthermore, the device 1 is easy and cheap to make and presents reduced dimensions such as to possibly permit an easy integration inside the electrical load 5. In particular, the extreme building simplicity of the device 1 make it possible to build it by means of different techniques, such as the printed circuit technique, the integrated circuit technology or the nanotechnology.

## Claims

1. A device for modifying and rebalancing ionisation for an electrical load (5), which is powered by an electrical power source (3); the device (1) being **characterised in that** it comprises at least one pair of electrical conductive elements (6) configured so as to be crossed, in use, by at least one part of an electrical current (IA) absorbed by the electrical load (5), mutually crossingly arranged and electrically insulated one with respect to the other.

2. A device according to Claim 1, comprising a pair of inputs (2) and a pair of outputs (4); the pair of inputs (2) being designed to be connected to said electrical power source (3) and the pair of outputs (4) being designed to be connected to said electrical load (5).

3. A device according to Claim 2, in which each electrical conductive element (6) is extended between an input (2) of said pair of inputs (2) and a respective output (4) of said pair of outputs (4).

4. A device according to any one of the preceding Claims, in which said electrical conductive elements (6) present a straight shape.

5. A device according to any one of the preceding Claims, comprising a plurality of said pairs of electrical conductive elements (6) mutually cascadingly connected such that each of said pairs is crossed by said part of the electrical current (IA) absorbed by said electrical load (5).

6. A device according to any one of the Claims from 1 to 4, comprising a plurality of said pairs of electrical conductive elements (6) mutually electrically connected in parallel such that each of said pairs is crossed by a respective fraction of said part of the electrical current (IA) absorbed by said electrical load (5).

7. A device according to Claim 6, in which said pairs of electrical conductive elements (6) are arranged on respective mutually parallel planes (8).

8. A device according to any one of the Claims from 5 to 7, in which said electrical conductive elements (6) present a straight shape; the two electrical conductive elements (6) of each of said pairs of electrical conductive elements (6) defining an angle; the angle of each pair of electrical conductive elements (6) being different from the angle of any other pair of electrical conductive elements (6).

9. A device according to any one of the preceding Claims, in which said electrical conductive elements (6) are made of a same electrical conductive material.

10. A device according to any one of the Claims from 1 to 8, in which each of said pairs of electrical conductive elements (6) comprises a first electrical conductive element (6) and a second electrical conductive element (6); the first and the second electrical conductive elements (6) are each made of a respective electrical conductive material; the electrical conductive material of the first electrical conductive element (6) being different from the electrical conductive material of the second electrical conductive element (6).

11. A device according to Claim 9 or 10, in which said electrical conductive material is chosen from a group of materials consisting in:
- metal;
- metallic alloy;
- conducting rubber; and
- superconductor material.

12. A device according to any one of the Claims from 2 to 11, comprising at least one electrical filter (7) arranged between said pair of inputs (2) and said pairs of electrical conductive elements (6).

13. A device according to any one of the Claims from 2 to 11, comprising at least one electrical filter (7) arranged between said pairs of electrical conductive elements (6) and said pair of outputs (4).

14. A device according to Claim 12 or 13, in which said electrical filter (7) is of the passive type.

15. A device according to Claim 14, in which said electrical filter (7) of the passive type comprises a double-n passband filter.

16. A device according to Claim 12 or 13, in which said electrical filter (7) is of the active type.

17. A device according to any one of the preceding Claims, and built by means of printed circuit technique.

18. A device, according to any one of the Claims from 1 to 16, and built by means of miniaturisation techniques.

19. An electrical load (5) provided with a device for modifying and rebalancing ionisation (1) according to any one of the preceding Claims.
